# NEW EUROPEAN PATENT SPECIFICATION

(11) **EP 2 658 514 B2**
(45) Date of publication and mention of the opposition decision: **24.11.2021**
(45) Mention of the grant of the patent: 24.04.2019
(21) Application number: 11815697.5
(22) Date of filing: 27.12.2011
(51) Int. Cl.: A61Q 5/02, A61K 8/39, A61K 8/44, A61K 8/46, A61K 8/60, A61K 8/41, A61K 8/49, A61Q 5/12, A61Q 5/06, A61Q 5/00

(54) **CLEANSING COMPOSITION ESPECIALLY FOR ARTIFICIALLY COLOURED HAIR**
REINIGUNGSZUSAMMENSETZUNG, INSBESONDERE FÜR KÜNSTLICH GEFÄRBTES HAAR
COMPOSITION DE NETTOYAGE EN PARTICULIER POUR DES CHEVEUX ARTIFICIELLEMENT COLORÉS

(30) Priority: 28.12.2010 EP 10197193
(43) Date of publication of application: 06.11.2013
(73) Proprietor: Kao Germany GmbH, 64297 Darmstadt (DE)
(72) Inventor: HOFFMANN, Martin, 64673 Zwingenberg (DE); OLSSON, Kristin, 68523 Ladenburg (DE)
(74) Representative: Grit, Mustafa
(86) International application number: PCT/EP2011/074067
(87) International publication number: WO 2012/089719

(56) References cited:
- EP-A1- 1 925 290
- EP-A1- 2 110 119
- EP-A1- 2 161 017
- EP-A1- 2 161 018
- EP-A1- 2 184 051
- EP-A1- 2 194 096
- EP-A1- 2 196 186
- EP-A1- 2 204 160
- DE-C1- 19 737 604
- DATABASE GNPD [Online] Mintel; October 2010 (2010-10), "Refreshing Shampoo, Conditioner & Body wash", XP002637085, Database accession no. 1407678 & US 5 756 436 A (ROYCE DOUGLAS ALLAN [US] ET AL) 26 May 1998 (1998-05-26) & US 5 837 661 A (EVANS MARK DAVID [US] ET AL) 17 November 1998 (1998-11-17)

## Description

The present invention relates to an aqueous cleansing composition comprising at least three anionic surfactants and a non-ionic surfactant providing exceptionally good colour stability for artificially coloured human hair.

Cleansing compositions have been known for many years. Many patent applications and scientific publications deal with such compositions aiming at cleansing, especially improved conditioning effects after cleansing. Cleansing compositions wash out dirt on the hair and at the same time dyestuffs deposited onto or into hair are also washed out, which leads to shorter colour durability after coloration services and is not economical in terms of frequent hair dressing need and also health of hair. Attempts have been made to reduce colour wash out from coloured hair with various ways. One of them is so called colour sealing which requires application of an additional mostly hydrophobic composition right after colouring hair so that the dyes are not easily solubilised by the high surfactant content of cleansing compositions. The other approach has been to use a special surfactant combination which interacts with dyestuff molecules and also with hair in a lesser extent. None of the approaches are optimal for variably coloured hair and, therefore, there is a great need for further improvements in the field.

Aim of the present invention is to provide an aqueous cleansing composition having optimal benefits in terms of foam properties measured with its volume and creaminess as well as improved conditioning effects on keratin fibres, especially human hair, providing optimized combability, smoothness, elasticity, softness, volume and body and at the same time washes out artificial hair colour in a lesser extend so that the coloured hair keeps its colour for a longer time and therefore its shine and healthy appearance.

Present inventors have surprisingly found that an aqueous cleansing composition comprising three anionic surfactants and a non-ionic surfactant and free from amphoteric surfactants provides excellent foam and conditioning properties and also washes out less colour from hair so that long lasting colours are achieved. Accordingly, the first object of the present invention is an aqueous cleansing composition comprising three anionic surfactants and an alkyl polyglucoside as a nonionic surfactant wherein it is free of amphoteric surfactants and the total surfactant concentration is in the range of 7.5 to 50% by weight calculated to the total of the composition.

The second object of the present invention is the use of the aqueous cleansing composition of the present invention for cleansing hair especially artificially coloured hair.

The fourth object of the present invention is kit for treating hair comprising the cleansing composition of the present invention and optionally at least one composition comprising a hair dye.

With the term " free of amphoteric surfactant" it is meant that the compositions actually do not comprise amphoteric surfactant but small quantities may be comprised which does not disturb in solving the problem solved with the present invention.

Compositions of the present invention comprise surfactants at a total concentration of 7.5 to 50% by weight calculated to the total of the composition. Preferably the total surfactant concentration is in the range of 7.5 to 30% and most preferably 10 to 30% and in particular 10 to 25% by weight (all values are included in the given ranges) calculated to the total of the composition. Compositions comprise anionic surfactants in the range of 50 to 95% by weight of the total surfactants. Preferably the total anionic surfactant concentration is in the range of 60 to 95%, more preferably 70 to 95% and most preferably 75 to 95% by weight of the total surfactants.

Cleansing compositions of the present invention comprises three anionic surfactants.

The first surfactant is an alkyl ether sulphate surfactant according to the following general structure

R₁(OCH₂CH₂)ₙOSO₃M

wherein R₁ is a saturated or unsaturated, branched or straight alkyl chain with 10 to 18C atoms, n is a number between 1 and 5 (the values are included) and M is a monovalent cation preferably selected from ammonium, sodium and potassium. In the most preferred from of the present invention R₁ is lauryl, n is on average between 1.5 and 3 (the values are included) and M is sodium.

Suitable sulphate surfactants according to the above general structure are laureth, myreth or coceth sulphates or their salts such as sodium, potassium or ammonium salts. Most preferred are sodium laureth sulphates with 1.5 to 3 ethoxy units on average.

Concentration of the first sulphate surfactant is in the range of 20 to 80%, preferably 30 to 70%, more preferably 40 to 70% and most preferably 45 to 60% by weight of the total anionic surfactants in the compositions of the present invention.

The second and the third anionic surfactants are amino acid surfactants and selected from the following general structure 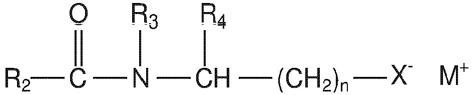 wherein R₂ is a saturated or unsaturated, straight or branched alkyl chain with 7 to 17 C atoms, R₃ is H or a methyl, R₄ is H, COO⁻M⁺, CH₂COO⁻ M or COOH, n is 0 to 2, X is COO⁻ or SO₃⁻ and M is independent from each other H, sodium, potassium or ammonium.

With the term amino acid surfactants especially those surfactants are meant derived from taurate, glutamate, alanin or alaninate, sarcosinate and aspartate.

In the preferred form of the present invention the second anionic surfactant is a sarcosinate surfactant according to the general formula 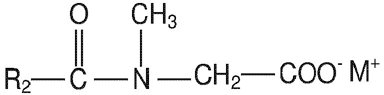 wherein R₂ is a saturated or unsaturated, straight or branched alkyl chain with 7 to 17 C atoms, and more preferably 9 to 13 C atoms, and M is H, sodium, potassium or ammonium.

Suitable examples are potassium lauroyl sarcosinate, potassium cocoyl sarcosinate, sodium cocoyl sarcosinate, sodium lauroyl sarcosinate, sodium myristoyl sarcosinate, and sodium palmitoyl sarcosinate and mixtures thereof. Preferred are potassium lauroyl sarcosinate, potassium cocoyl sarcosinate, sodium cocoyl sarcosinate, and sodium lauroyl sarcosinate and mixtures thereof. More preferred are sodium cocoyl sarcosinate, and sodium lauroyl sarcosinate and mixtures thereof.

In a further preferred form of the present invention the third anionic surfactant is a glutamate surfactant according to the general formula 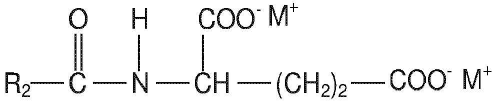 wherein R₂ is a saturated or unsaturated, straight or branched alkyl chain with 7 to 17 C atoms, and more preferably 9 to 13 C atoms, and M is independent from each other H, sodium, potassium or ammonium.

Suitable examples are dipotassium capryloyl glutamate, dipotassium undecylenoyl glutamate, disodium capryloyl glutamate, disodium cocoyl glutamate, disodium lauroyl glutamate, disodium stearoyl glutamate, disodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, and sodium undecylenoyl glutamate and mixtures thereof. Preferred are disodium capryloyl glutamate, disodium cocoyl glutamate, disodium lauroyl glutamate, potassium capryloyl glutamate, potassium cocoyl glutamate, potassium lauroyl glutamate, potassium myristoyl glutamate, sodium capryloyl glutamate, sodium cocoyl glutamate, sodium lauroyl glutamate, and sodium myristoyl glutamate and mixtures thereof. More preferred are disodium capryloyl glutamate, disodium cocoyl glutamate, disodium lauroyl glutamate, potassium capryloyl glutamate, potassium cocoyl glutamate, potassium lauroyl glutamate, sodium capryloyl glutamate, sodium cocoyl glutamate, and sodium lauroyl glutamate and mixtures thereof. Most preferred are potassium cocoyl glutamate, potassium lauroyl glutamate, sodium cocoyl glutamate, and sodium lauroyl glutamate. Sodium cocoyl and lauroyl glutamates are particularly preferred.

In the preferred form of the present invention the second and the third anionic surfactants particularly sarcosinate and glutamate surfactants are comprised in the compositions at a weight ratio of the sarcosinate to glutamate surfactant in the range of 1:1 to 10:1, preferably 1:1 to 8:1 and more preferably 1:1 to 6:1 and most preferably 1:1 to 5:1 and in particular in the range of 3:1 to 4:1.

Compositions of the present invention comprise an alkyl polyglucoside as a non-ionic surfactant. Suitable are alkyl (poly)glucosides according to the general formula

R₈-O-(R₉O)ₙ-Zₓ,

wherein R₈ is an alkyl group with 8 to 18 carbon atoms, R₉ is an ethylene or propylene group, Z is a saccharide group with 5 to 6 carbon atoms, n is a number from 0 to 10 and x is a number between 1 and 5.

Suitable non-limiting examples are caprylyl glucoside, decyl glucoside, lauryl glucoside, myrystyl glucoside and cocoyl glucoside. Preferred are decyl glucoside, lauryl glucoside and cocoyl glucoside.

In a further preferred embodiment of the present invention, aqueous cleansing composition consists of three anionic surfactants preferably a sulphate surfactant, a sarcosinate surfactant and a glutamate surfactant and a non-ionic surfactant preferably an alkyl (poly)glucoside.

Nonionic surfactants are comprised in the cleansing compositions of the present invention at a concentration of 5 to 80% by weight of the total surfactants. Preferably the total nonionic surfactant concentration is in the range of 5 to 70%, more preferably 5 to 60% and most preferably 5 to 50% by weight of the total surfactants.

Compositions of the present invention may comprise additional surfactants known generally in the cleansing compositions excluding the amphoteric ones as the compositions are substantially free of amphoteric surfactants.

Additional anionic surfactants may be α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates and carboxylates such as alkyl polyether carboxylic acids and the salts thereof of the formula

R₇-(C₂H₄O)ₙ-O-CH₂COOX,

wherein R₇ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 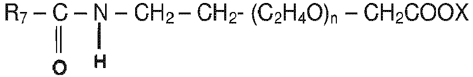 wherein R and X have the above meanings, and n is in particular a number from 1 to 10, preferably 2.5 to 5. Also worth to mention are C₈-C₂₀-acyl isethionates, alone or in admixture with other anionic surfactants, as well as sulfofatty acids and the esters thereof.

Further anionic surfactants may be taurate surfactants, alanine or alaninate surfactants, glycine surfactants and aspartate surfactants.

Taurate surfactants to mention are according to the general formula 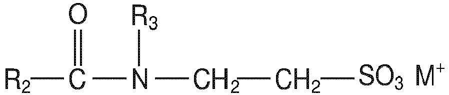 wherein R₂ is a saturated or unsaturated, straight or branched alkyl chain with 7 to 17 C atoms, and more preferably 9 to 13 C atoms, R₃ is H or methyl, and M is H, sodium or potassium. Nonlimiting examples are potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, and sodium methyl stearoyl taurate and mixtures thereof.

Alanine or alaninate surfactants to mention are according to the general formula 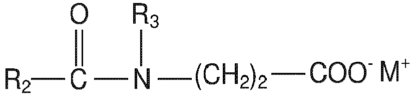 wherein R₁ is preferably a saturated or unsaturated, straight or branched alkyl chain with 7 to 17 C atoms, and more preferably 9 to 13 C atoms, R₂ is H or methyl and M is H, sodium or potassium. Nonlimiting examples are cocoyl methyl β-alanine, lauroyl β-alanine, lauroyl methyl β-alanine, myristoyl β-alanine, potassium lauroyl methyl β-alanine, sodium cocoyl alaninate, sodium cocoyl methyl β-alanine and sodium myristoyl methyl β- alanine and mixtures thereof.

Glycine surfactants to mention are according to the general formula 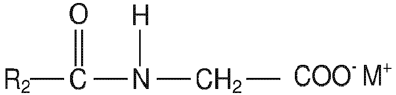 wherein R₂ is preferably a saturated or unsaturated, straight or branched alkyl chain with 7 to 17 C atoms, and more preferably 9 to 13 C atoms, and M is H, sodium or potassium. Nonlimiting examples are palmitoyl glycine, sodium lauroyl glycinate, sodium cocoyl glycinate, sodium myristoyl glycinate, potassium lauroyl glycinate, and potassium cocoyl glycinate and mixtures thereof.

Aspartate surfactants to mention are according to the general formula 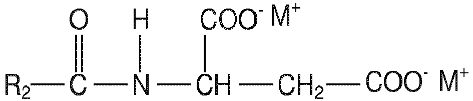 wherein R₂ is a saturated or unsaturated, straight or branched alkyl chain with 7 to 17 C atoms, and more preferably 9 to 13 C atoms, and M is independent from each other H, sodium, potassium or ammonium. Nonlimiting examples are sodium lauroyl aspartate, sodium myristoyl aspartate, sodium cocoyl aspartate, sodium caproyl aspartate, disodium lauroyl aspartate, disodium myristoyl aspartate, disodium cocoyl aspartate, disodium caproyl aspartate, potassium lauroyl aspartate, potassium myristoyl aspartate, potassium cocoyl aspartate, potassium caproyl aspartate, dipotassium lauroyl aspartate, dipotassium myristoyl aspartate, dipotassium cocoyl aspartate, and dipotassium caproyl aspartate and mixtures thereof.

Nonionic surfactants may be comprised in the cleansing compositions of the present invention, are such as long-chain fatty acid dialkanolamides, namely coco fatty acid diethanolamide and myristic fatty acid diethanolamide, various sorbitan esters, such as polyethylene glycol sorbitan stearic acid ester, fatty acid polyglycol esters or polycondensates of ethyleneoxide and propyleneoxide, as they are on the market, for example, under the trade name "Pluronics^{R}", as well as fatty alcohol ethoxylates, amineoxides such as C₁₂-C₁₈- alkyl dimethyl amineoxides namely lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain as such on the market, for example, under the trade names "Ammonyx®", "Aromox®" or "Genaminox®" and also C₁₀-C₂₂-fatty alcohol ethoxylate, especially C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the INCI nomenclature, including addition of the number of ethylene oxide molecules, e.g., "Laureth-16" with the average degree of ethoxylation thereby ranges between about 2.5 and about 25, preferably about 10 and about 20.

In a further preferred embodiment, cleansing composition of the present invention comprises hair-conditioning agents. Conditioning agents can be selected from oily substances, non-ionic substances, cationic amphiphilic ingredients, cationic polymers other than the one described above or their mixtures.

Oily substances are selected from such as silicone oils, either volatile or non-volatile, natural and synthetic oils. Among silicone oils those can be added to the compositions include dimethicone, dimethiconol, polydimethylsiloxane, DC fluid ranges from Dow Corning, arylated silicones such as phenyl trimethicone or any other silicone with up to 5 aryl, preferably phenyl, group in its molecule such as trimethyl pentaphenyl trisiloxane, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents can be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁₁CO(OCH₂CH₂)ₙOH

or

R₁₁CO(OCH₂CH₂)ₙOOCR₁₂

where R₁₁ and R₁₂ are independent from each other saturated, unsaturated or branched or non-branched alkyl chain with 7 to 21 C atoms and n is typically 2 - 100.

Composition of the present invention comprises preferably at least one cationic polymer as a conditioning agent. Cationic polymers are comprised in the compositions at a concentration of 0.01 to 5%, preferably 0.02 to 4%, more preferably 0.05 to 3% and most preferably 0.1 to 2.5% by weight calculated to the total the composition. Suitable cationic polymers are of best known with their INCI category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum an its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 67, polyquaternium 87 and polyquaternium 70.

The cationic polymers also include the quaternized products of graft polymers from organopolysiloxanes and polyethyl oxazolines described in EP-A 524 612 and EP-A 640 643.

Although less preferred, cleansing compositions of the present invention may comprise additionally one or more cationic surfactant(s) as conditioner presented with the general formula 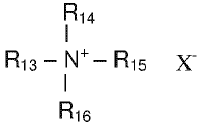 where R₁₃ is a saturated or unsaturated, branched or non-branched alkyl chain with 8-24 C atoms or

R₁₇CONH(CH₂)ₙ

where R₁₇ is saturated or unsaturated, branched or non-branched alkyl chain with 7-21 C atoms and n has value of 1 - 4, or

R₁₈COO(CH₂)ₙ

where R₁₈ is saturated or unsaturated, branched or non-branched alkyl chain with 7-21 C atoms and n has value of 1 - 4, and
R₁₄ is unsaturated or saturated, branched or non-branched alkyl chain with 1 - 24 C atoms or

R₁₇CONH(CH₂)ₙ

or

R₁₈COO(CH₂)ₙ

where R₁₇, R₁₈ and n are same as above.

R₁₅ and R₁₆ are lower alkyl chain with 1 to 4 carbon atoms which may be substituted with one or more hydroxyl groups, and X is anion such as chloride, bromide, methosulfate.

Typical examples of those ingredients are cetyl trimethly ammonium chloride, steartrimonium chloride, behentrimonium chloride, stearamidopropyl trimonuim chloride, palmitamidopropyltrimonium chloride, dioleoylethyl dimethyl ammonium methosulfate, dioleoylethyl hydroxyethylmonium methosulfate.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, silicone oil and derivatives and cationic surfactants is in the range of 0.01 to 5% by weight, preferably 0.01 to 3.5% by weight, more preferably 0.05 to 2.5% and most preferably 0.1 to 1.5% by weight calculated to the total composition. Most preferred conditioning agents are cationic polymers.

In another preferred form of the invention, aqueous cleansing composition comprises at least one organic solvent such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, polypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzyl alcohol and polypropylene glycols. Total concentration of organic solvents in the shampoo composition should not exceed 5% by weight, preferably in the range of 0.1 to 3%, more preferably 0.5 to 2.5% by weight calculated to total composition.

Further conditioning additives are hair conditioning and/or styling polymers. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in conditioning shampoo composition of the present invention. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer®"; copolymers from methacryl oylethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl aminoalkyl (meth)acrylamides containing basic groups, in particular amino groups; copolymers from N-octyl acrylamide, methyl methacrylate, hydroxypropyl methacrylate, N-tert.-butyl aminoethyl - methacrylate and acrylic acid, as well as the copolymers known from US-A 3,927,199, are applicable.

Aqueous cleansing composition of the present invention may comprise at least one glyceryl ether of the following formula 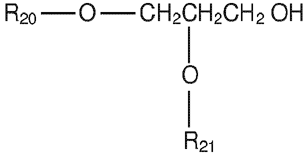 wherein R₂₀ is straight or branched, saturated or unsaturated alkyl chain with 4 to 24 C atoms, preferably 4 to 18 and more preferably 4 to 12 C atoms and R₂₁ is H, or straight or branched, saturated or unsaturated alkyl chain with 4 to 24 C atoms, 4 to 18 and more preferably 4 to 12 C atoms and most preferably R₅ is H, at a concentration of 0.1 to 15%, preferably 0.1 to 10% and more preferably 0.25 to 7.5% and most preferably 0.5 to 5% by weight calculated to total composition.

Suitable unlimited examples are glyceryl butyl ether, glyceryl isobutyl ether, glyceryl tert-butyl ether, glyceryl pentyl ether, glyceryl isopentyl ether, glyceryl hexyl ether, glyceryl isohexyl ether, glyceryl heptyl ether, glyceryl octyl ether, glyceryl ethylhexyl ether, glyceryl nonyl ether, glyceryl decyl ether, glyceryl isodecyl ether, glyceryl lauryl ether, glyceryl myristyl ether, glyceryl palmityl ether, glyceryl stearyl ether and glyceryl behenyl ether and their mixtures. Most preferred are glyceryl butyl ether, glyceryl isobutyl ether, glyceryl tert-butyl ether, glyceryl pentyl ether, glyceryl isopentyl ether, glyceryl hexyl ether, glyceryl isohexyl ether, glyceryl heptyl ether, glyceryl octyl ether, glyceryl ethylhexyl ether, glyceryl nonyl ether, glyceryl decyl ether, glyceryl isodecyl ether are glyceryl lauryl ether, and their mixtures.

It should be noted that within the disclosure of the present description, glyceryl decyl ether is used as synonym of decyl glycerine. For the other compounds in the above paragraph the same is valid.

Aqueous cleansing composition of the present invention may further comprise at least one fatty alcohol of the following formula

R₂₅-OH

wherein R₂₅ is straight or branched, saturated or unsaturated alkyl chain with 8 to 24, preferably 10 to 22, more preferably 12 to 18 and most preferably 12 to 16C atoms at a concentration of 0.1 to 5%, preferably 0.1 to 4% and more preferably 0.25 to 3% and most preferably 0.5 to 2.5% by weight calculated to total composition.

Suitable non-limiting preferred examples are decyl alcohol, myristyl alcohol, lauryl alcohol, cetyl alcohol, stearyl alcohol, behenyl alcohol, and arachidyl alcohol and their mixtures. More preferred are decyl alcohol, myristyl alcohol, lauryl alcohol, cetyl alcohol, and stearyl alcohol. Most preferred are decyl alcohol, myristyl alcohol and lauryl alcohol, and their mixtures.

Cleansing composition of the present invention is preferably pearly. Pearl-shiny appearance is achieved with those dispersed in cleansing conditioning compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kinds of mixtures are available commercially.

Solubilizers may be added to the compositions especially when oily substances are chosen as conditioning agents and fragrance oils with highly lipophilic properties. Typical solubilizers may be hydrogenated castor oil known with the trade mark Cremophor CO series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to total composition.

The cleansing composition may contain active ingredients selected from UV filters, moisturisers, sequestering agents, and natural ingredients.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are preferably selected from polycarboxy acids. The preferred one is ethylene diamine tetraacetic acid, EDTA. Typical useful concentration range for sequestering agents is of 0.01 - 2.5% by weight calculated to the total composition.

The UV filters are that oil and water soluble ones for the purpose of protecting hair and hair colour. In other words, anionic and non-ionic, oily, UV filters are suitably used in the compositions of the present invention. Suitable UV-absorbing substances is are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene) -DL-campher, and/or polysilicone-15. The amount of the UV-absorber ranges typically from about 0.01 % to 2.5%, more preferably from 0.05 % to 1 % by weight, calculated to the total composition.

Natural plant extracts are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, various "Extrapone®" products, and "Herbasol^{R}". Extracts and the preparation thereof are also described in "Hagers Handbuch der pharmazeutischen Praxis", 4th Ed..

Compositions of the present invention may comprise further at least one compound according to the formula 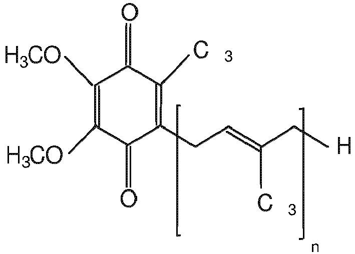 where n is a number from 1 to 10.

The compounds of the above formula are known as Ubiquinone, and also are known as Coenzyme. It should be noted that the compositions of the present invention can certainly comprise more than one ubiqinone. Preferred ubiqinones are the ones where n is a number between 6 and 10 and especially preferred is Ubiqinone 50 where n is 10, also known as Coenzyme Q10. Concentration ubiqinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Cleansing compositions of the present invention can also comprise synthetic mica as a further shine enhancer.

Suitable metal oxide or oxides for coating synthetic mica are titanium dioxide, chromium oxide, ferric oxide or mixtures thereof. In the present invention the preferred is synthetic mice coated with titanium dioxide. Such materials are commercially available from Sun Chemical Corporation and known with their INCI names Synthetic Fluorphologopite.

The particle size distribution of synthetic mica coated with a metal oxide or oxides is in the range of 1 to 750 µm, preferably 1 to 250 µm, more preferably 1 to 100 µm and most preferably 20 to 95 µm. The particle sizes referred are relating to the volume particle size distribution meaning that particles found in the coated synthetic mica having volume particle size in the given ranges.

Concentration of synthetic mica coated with at least metal oxide or oxides is from 0.001 to 10%, preferably 0.05 to 7.5%, more preferably 0.1 to 5% and most preferably 0.20 to 2.5% by weight calculated to total composition.

Further in a preferred embodiment of the present invention, compositions comprise at least one direct dye and as well deposit dyestuffs on the hair. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye. Certainly the combination of all three dyestuff categories is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87, and mixtures thereof

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium, and mixtures thereof.

Among those, the preferred anionic dyestuffs are Acid Red 52, Acid Violet 2, Acid Red 33, Acid Orange 4, Acid Red 27 and Acid Yellow 10 and their salts, and mixtures thereof. The most preferred anionic dyes are Acid Red 52, Acid Violet 2, Acid Red 33, Acid Orange 4 and Acid Yellow 10, and their salts, and mixtures thereof.

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid, and mixtures thereof.

Concentration of one or more direct dyes in total is in the range of 0.001 to 5% by weight, preferably 0.01 to 4% more preferably 0.05 to 3% and most preferably 0.1 to 2.5% by weight calculated to total composition. The most preferred among the direct dyes is cationic direct dyes.

It is self-understood that the shampoos according to the invention may comprise other substances customarily used in such compositions such as preservatives, fragrances.

The pH of the compositions according to the present invention is suitably between 2 and 8.0, preferably in the range of 2.5 to 7.0, more preferably 3 to 6.5 and most preferably 4 to 6.0 measured at ambient temperature with a suitable pH meter.

pH of the compositions is adjusted with acidic and alkaline compounds. Acidic compounds can be inorganic and organic acid or their mixtures. Nonlimiting suitable examples are citric acid, lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Alkaline compounds such as sodium hydroxide can be used to adjust the pH of the compositions.

Aqueous cleansing composition of the present invention preferably comprises one or more thickeners. Suitable ones are ethoxylated polyglyceryl esters with total ethoxy units in the range of 50 to 200 and fatty acyl chain length of 8 to 22 C atoms such as PEG-80 glyceryl cocoate, PEG-90 glyceryl isostearate, PEG-120 glyceryl stearate, PEG-200 glyceryl stearate, PEG-80 glyceryl tallowate, PEG-82 glyceryl tallowate, PEG-130 glyceryl tallowate, and PEG-200 glyceryl tallowate, PEG-18 gylceryl oleate/cocoate, PEG-160 sorbitan triisostearate and inorganic salt in particular sodium chloride when especially composition comprise alkyl ether sulphate type of surfactants.

Cleansing compositions of the present invention preferably has a viscosity in the range of 500 to 20,000 mPa.s, more preferably 1,000 to 15,000 mPa.s and most preferably 1,500 to 10,000 mPa.s measured at 20°C with a Brookfield viscosimetre using fro example Spindle 5 at appropriate rotation speed.

The following examples are to illustrate the invention, but not to limit. The products according to the invention are prepared by mixing the individual components in water, whereby it is also possible to use pre-mixtures of various ingredients.

### Example 1

**Table I: Comparative aqueous cleansing compositions**

| | % by weight | | | | |
|---|---|---|---|---|---|
| | A | B | C | D | E |
| Sodium laureth sulfate | 20 | 17 | 17 | 17 | 12 |
| Sodium lauryl sarcosinate | - | - | 3 | - | 5 |
| Coco glucoside | - | 3 | - | - | 1.5 |
| Sodium lauroyl glutamate | - | - | - | 3 | 1.5 |
| Citric acid | q.s to pH | | | | |
| | 5.0 | | | | |
| Preservative, fragrance | q.s. | | | | |
| Water | q.s. to 100 | | | | |
| Shampoo composition E is according to the invention and A to D represents comparative compositions. | | | | | |

Hair tresses were coloured with a commercially available oxidative colouring composition comprising oxidative and direct dyes. The tresses so coloured were washed 10 times with above compositions and before starting the test and afterwards L, a and b values were measured. With the help of the known equation ΔE values were calculated to present colour difference numerically.

Results are presented in Table II.

**Table II: Results of colour wash out test.**

| | ΔE value |
|---|---|
| Composition A | 10.2 |
| Composition B | 8.8 |
| Composition C | 9.2 |
| Composition D | 8.6 |
| Composition E | 7.9 |

It should be noted that the higher the ΔE value the larger the colour difference between the two tresses. In the opposite, the smaller the ΔE value the smaller the colour difference between the two tresses. From the above results, it is clear that cleansing composition according to the invention produces the lowest ΔE value. Similar effects were observed with the following compositions.

### Example 2

| | % by weight |
|---|---|
| Sodium lauryl ether sulphate | 10.6 |
| Sodium lauroyl glutamate | 1.5 |
| Sodium lauroyl sarcosinate | 5.0 |
| Decyl glucoside | 2.0 |
| Polyquaternium-7 | 0.5 |
| PEG-90 glyceryl isostearate | 3.0 |
| Citric acid/sodium hydroxide | q.s. to pH 5.5 |
| Preservative, fragrance | q.s |
| Water | to 100 |

The above shampoo was judged to have rich and creamy foam in a monadic test by the volunteers. It was furthermore mentioned that it foams very quickly. Additionally, the hair washed was excellently combable and had good shine.

### Example 3

| | % by weight |
|---|---|
| Sodium lauryl ether sulphate | 9.9 |
| Cocyl glucoside | 3.0 |
| Sodium lauroyl glutamate | 1.5 |
| Sodium lauroyl sarcosinate | 5.0 |
| Polyquaternium-10 | 1.0 |
| PEG-18 Glyceryl oleate/cocoate | 1.0 |
| Citric acid/sodium hydroxide | q.s. to pH 5.5 |
| Preservative, fragrance | q.s |
| Water | to 100 |

The above composition has excellent creamy rich foam and conditions hair excellently in terms of combability and soft hair feeling.

### Example 4

| | % by weight |
|---|---|
| Sodium lauryl ether sulfate | 9.0 |
| Cocyl glucoside | 3.0 |
| Sodium lauroyl sarcosinate | 5.0 |
| Sodium lauroyl glutamate | 1.5 |
| Ethylhexyl glycerine | 1.5 |
| Lauryl alcohol | 0.7 |
| Polyquaternium-10 | 0.5 |
| Dimethicone | 0.5 |
| Ubiquinone | 0.1 |
| Sodium chloride | 1.2 |
| PPG-9 | 2.0 |
| Citric acid/sodium hydroxide | q.s. to pH 5.0 |
| Preservative, fragrance | q.s |
| Water | to 100 |

The above composition improves hair volume, gives hair more elasticity in addition to the excellent creamy foam and conditioning effect in terms of combability, shine and soft hair feeling. Colour wash out effect was observed to be excellent.

### Example 5

| | % by weight |
|---|---|
| Sodium lauryl ether sulphate | 9.9 |
| Cocoyl polyglucoside | 2.5 |
| Sodium lauroyl sarcosinate | 4.0 |
| Sodium cocoyl glutamate | 1.0 |
| Decyl glycerine | 1.0 |
| Decyl alcohol | 1.0 |
| Polyquaternium-16 | 0.8 |
| Dimethicone | 0.5 |
| PEG-160 sorbitan triisostearate | 1.0 |
| PPG-9 | 1.2 |
| Basic red 51 | 0.1 |
| Citric acid/sodium hydroxide | q.s. to pH 5.5 |
| Preservative, fragrance | q.s |
| Water | to 100 |

The above composition gives hair a red shine, and additionally delivers excellent conditioning effect in terms of more elasticity, combability, shine and soft hair feeling in addition to the excellent creamy rich foam. The composition foams very quickly.

### Example 6

| | % by weight |
|---|---|
| Sodium lauryl ether sulfate | 9.0 |
| Decyl glucoside | 3.0 |
| Sodium lauroyl sarcosinate | 6.0 |
| Sodium cocoyl glutamate | 2.0 |
| Guarhydroxypropyltrimonium chloride | 0.2 |
| Polyqauternium-7 | 0.2 |
| Sodium chloride | 1.0 |
| Heptyl glycerine | 0.7 |
| Myristyl alcohol | 0.5 |
| PPG-9 | 1.0 |
| Trimethyl pentaphenyl trisiloxane | 0.3 |
| Basic yellow 87 | 0.08 |
| Basic red 76 | 0.001 |
| Citric acid/sodium hydroxide | q.s. to pH 5.0 |
| Preservative, fragrance | q.s |
| Water | to 100 |

Excellent conditioning effects were observed in terms of volume, combability, elasticity and managabiliy and additionally an excellent golden blonde shine was observed on light blond hair. Excellent foam quality in terms of speed, volume and creaminess was observed in a monadic test.

### Example 7

| | % by weight |
|---|---|
| Sodium lauryl ether sulfate | 9.0 |
| Cocyl glucoside | 3.0 |
| Sodium cocoyl sarcosinate | 6.0 |
| Sodium lauroyl glutamate | 2.0 |
| Polyqauternium-6 | 1.0 |
| PEG-80 glyceryl oleate/cocoate | 1.0 |
| Ethylhexyl glycerine | 1.0 |
| Myristyl alcohol | 1.0 |
| PPG-9 | 1.0 |
| Trimethyl pentaphenyl trisiloxane | 0.3 |
| Basic red 51 | 0.1 |
| Basic orange 31 | 0.05 |
| Citric acid/sodium hydroxide | q.s. to pH 5.0 |
| Preservative, fragrance | q.s |
| Water | to 100 |

Excellent red shine were observed on medium blond hair, in addition to excellent foam characteristics in terms of speed, volume and creaminess in a monadic test.

### Example 8

| | % by weight |
|---|---|
| Sodium lauryl ether sulfate | 9.0 |
| Decyl glucoside | 3.0 |
| Sodium lauroyl sarcosinate | 4.0 |
| Sodium cocoyl glutamate | 1.0 |
| Ethylhexyl glycerine | 1.0 |
| Myristyl alcohol | 1.0 |
| Polyquaternium-10 | 0.5 |
| PEG-90 glyceryl isostearate | 3.5 |
| PPG-9 | 0.7 |
| Carbopol Aqua CC | 5.0 |
| Synthetic fluorphologopite* | 0.5 |
| Citric acid/sodium hydroxide | q.s. to pH 4.7 |
| Preservative, fragrance | q.s |
| Water | to 100 |
| *: Synthetic fluorphologopite used is commercially available from Sun Chemical Corporation under the trade name SunShine Glitter White with a particle size distribution in the range of 20 to 95 µm. | |

The above composition delivered excellent volume and shine to dark blonde fine hair. Foam characteristics were found to be excellent in terms of volume, speed and creaminess in a monadic test.

### Example 9

| | % by weight |
|---|---|
| Sodium lauryl ether sulfate | 13.0 |
| Sodium lauroyl sarcosinate | 4.0 |
| Decyl glucoside | 1.5 |
| Sodium cocoyl glutamate | 1.0 |
| Ethylhexyl glycerine | 1.0 |
| Myristyl alcohol | 1.0 |
| Quaternium 80 | 0.5 |
| Polyquaternium-10 | 0.2 |
| Sodium chloride | 1.0 |
| PPG-9 | 1.7 |
| Citric acid/sodium hydroxide | q.s. to pH 5.0 |
| Preservative, fragrance | q.s |
| Water | to 100 |

Above shampoo was found to be excellent volume giving shampoo to fine hair in a monadic test in addition to the excellent foam characteristics as in the previous examples.

With the following examples similar results were found as in the previous examples in hair conditioning and foam characteristics.

### Example 10

| | % by weight |
|---|---|
| Sodium lauryl ether sulphate | 8.0 |
| Cocoyl polyglucoside | 3.0 |
| Sodium lauroyl sarcosinate | 6.0 |
| Ethylhexyl glycerine | 1.0 |
| Myristyl alcohol | 1.0 |
| Sodium cocoyl glutamate | 2.0 |
| Trimethyl pentaphenyl trisiloxane | 0.3 |
| Polyquaternium-7 | 0.5 |
| PEG-120 glyceryl stearate | 3.0 |
| PPG-15 | 1.7 |
| Citric acid/sodium hydroxide | q.s. to pH 5.2 |
| Preservative, fragrance | q.s |
| Water | to 100 |

### Example 11

| | % by weight |
|---|---|
| Sodium lauryl ether sulphate | 9.0 |
| Cocoyl glucoside | 5.0 |
| Sodium lauroyl sarcosinate | 4.0 |
| Sodium cocoyl glutamate | 1.2 |
| Ethylhexyl glycerine | 1.0 |
| Myristyl alcohol | 1.0 |
| Polyquaternium-7 | 1.0 |
| PEG-90 glyceryl isostearate | 1.5 |
| Citric acid/sodium hydroxide | q.s. to pH 5.2 |
| Preservative, fragrance | q.s |
| Water | to 100 |

### Example 12

| | % by weight |
|---|---|
| Sodium lauryl ether sulphate | 5.0 |
| Sodium lauryl ether carboxylate | 3.0 |
| Decly glucoside | 3.0 |
| Sodium lauroyl sarcosinate | 2.0 |
| Ethylhexyl glycerine | 1.0 |
| Myristyl alcohol | 1.0 |
| Sodium lauroyl glutamate | 0.6 |
| Polyquaternium-7 | 0.5 |
| Dimethicone | 0.5 |
| Ubiquinone | 0.1 |
| PEG-18 Glyceryl oleate/cocoate | 1.2 |
| PPG-9 | 0.8 |
| Citric acid/sodium hydroxide | q.s. to pH 5.0 |
| Preservative, fragrance | q.s |
| Water | to 100 |

### Example 13

| | % by weight |
|---|---|
| Sodium lauryl ether sulphate | 3.0 |
| Sodium lauryl ether carboxylate | 6.0 |
| Cocoyl polyglucoside | 0.8 |
| Sodium cocyl glutamate | 2.0 |
| Sodium lauroyl sarcosinate | 3.0 |
| Polyquaternium-87 | 0.8 |
| Dimethicone | 0.5 |
| PEG-90 glyceryl isostearate | 3.0 |
| Basic red 51 | 0.1 |
| Citric acid/sodium hydroxide | q.s. to pH 5.5 |
| Preservative, fragrance | q.s |
| Water | to 100 |

### Example 14

| | % by weight |
|---|---|
| Sodium lauryl ether sulfate | 9.9 |
| Decyl glucoside | 3.0 |
| Sodium lauroyl sarcosinate | 5.0 |
| Sodium lauroyl glutamate | 1.6 |
| Ethylhexyl glycerine | 1.0 |
| Polyquaternium-87 | 1.0 |
| Sodium chloride | 1.3 |
| PPG-20 | 0.8 |
| Trimethyl pentaphenyl trisiloxane | 0.2 |
| Basic yellow 87 | 0.10 |
| Basic red 76 | 0.01 |
| Citric acid/sodium hydroxide | q.s. to pH 6.0 |
| Preservative, fragrance | q.s |
| Water | to 100 |

Increase of volume and an excellent golden blonde shine was observed on light blond hair. Conditioning effect in terms of manageability and soft feeling upon touching is excellent.

### Example 15

| | % by weight |
|---|---|
| Sodium lauryl ether sulfate | 5.0 |
| Sodium lauryl ether carboxylate | 4.0 |
| Decyl glucoside | 3.0 |
| Sodium lauroyl sarcosinate | 6.0 |
| Sodium lauroyl glutamate | 2.0 |
| Polyqauternium-7 | 0.7 |
| Guarhydroxypropyltrimonium chloride | 1.0 |
| PEG-120 glyceryl stearate | 1.8 |
| Dimethicone | 1.0 |
| Basic red 51 | 0.1 |
| Basic orange 31 | 0.05 |
| Citric acid/sodium hydroxide | q.s. to pH 5.7 |
| Preservative, fragrance | q.s |
| Water | to 100 |

An excellent red shine were observed on medium blond hair.

### Example 16

| | % by weight |
|---|---|
| Sodium lauryl ether sulphate | 10.0 |
| Decyl glucoside | 3.0 |
| Sodium lauroyl sarcosinate | 6.0 |
| Sodium lauroyl glutamate | 1.8 |
| Polyquaternium-10 | 0.5 |
| Polysilicone-15 | 0.35 |
| Dimethicone | 0.5 |
| Ubiquinone | 0.1 |
| Sodium chloride | 1.0 |
| PPG-9 | 0.9 |
| Citric acid/sodium hydroxide | q.s. to pH 4.8 |
| Preservative, fragrance | q.s |
| Water | to 100 |

The above shampoo conditions hair excellently in terms of combability, softness, shine and elasticity and additionally gives fine hair excellent long lasting volume.

### Example 17

| | % by weight |
|---|---|
| Sodium lauryl ether sulphate | 6.0 |
| Sodium lauryl ether carboxylate | 4.0 |
| Cocoyl glucoside | 3.0 |
| Sodium cocoyl sarcosinate | 5.0 |
| Sodium lauroyl glutamate | 1.5 |
| Polyquaternium-7 | 0.5 |
| Benzophenone-4 | 0.5 |
| Dimethicone | 0.5 |
| Ubiquinone | 0.1 |
| PEG-18 glyceryl oleate/cocoate | 1.2 |
| Citric acid/sodium hydroxide | q.s. to pH 5.5 |
| Preservative, fragrance | q.s |
| Water | to 100 |

The above shampoo conditions hair excellently in terms of combability, shine, softness and elasticity and additionally gives fine hair excellent long lasting volume.

### Example 18

| | % by weight |
|---|---|
| Sodium lauryl ether sulphate | 11.0 |
| Decyl glucoside | 3.0 |
| Sodium lauroyl sarcosinate | 6.0 |
| Sodium cocoyl glutamate | 2.0 |
| Polyquaternium-87 | 0.5 |
| Decyl glycerine | 1.0 |
| Ethylhexyl methoxy cinnamate | 0.3 |
| Dimethicone | 0.5 |
| Ubiquinone | 0.1 |
| Sodium chloride | 0.9 |
| PPG-9 | 0.7 |
| Citric acid/sodium hydroxide | q.s. to pH 5.2 |
| Preservative, fragrance | q.s |
| Water | to 100 |

### Example 19

| | % by weight |
|---|---|
| Sodium lauryl ether sulphate | 10.0 |
| Cocyl glucoside | 5.0 |
| Sodium lauroyl sarcosinate | 4.0 |
| Ethylhexyl glycerine | 1.0 |
| Myristyl alcohol | 1.0 |
| Sodium lauroyl glutamate | 1.0 |
| Polyquaternium-87 | 0.5 |
| Benzophenone-3 | 0.4 |
| Dimethicone | 0.5 |
| Sodium chloride | 1.0 |
| PPG-9 | 0.5 |
| Citric acid/sodium hydroxide | q.s. to pH 5.4 |
| Preservative, fragrance | q.s |
| Water | to 100 |

## Claims

1. Aqueous cleansing composition for keratin fibres especially for human hair **characterised in that** it comprises three anionic surfactants wherein the first anionic surfactant is an alkyl ether sulphate surfactant according to the following general structure
R₁ (OCH₂CH₂)ₙ OSO₃ M
wherein R₁ is a saturated or unsaturated, branched or straight alkyl chain with 10 to 18C atoms, n is a number between 1 and 5 (the values are included) and M is a monovalent cation preferably selected from H, ammonium, sodium and potassium, and
the second and the third anionic surfactants are amino acid surfactants selected from the following general structure 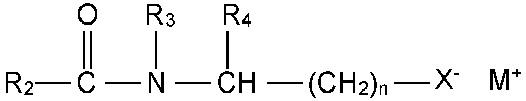
wherein R₂ is a saturated or unsaturated, straight or branched alkyl chain with 7 to 17 C atoms, R₃ is H or a methyl, R₄ is H, COO⁻ M⁺, CH₂COO⁻ M or COOH, n is 0 to 2, X is COO⁻ or SO₃⁻ and M is independent from each other H, sodium, potassium or ammonium, and an alkyl polyglucoside as
a non-ionic surfactant wherein it is free of amphoteric surfactants and the total surfactant concentration is in the range of 7.5 to 50% by weight calculated to the total of the composition.

2. The composition according to claim 1 **characterized in that** the first anionic surfactant is selected from sodium laureth sulphates with 1.5 to 3 ethoxy units on average.

3. The composition according to claims 1 and 2 **characterised in that** concentration of the first sulphate surfactant is in the range of 20 to 80% by weight calculated to the total of the anionic surfactants.

4. The composition according to any of the preceding claims **characterized in that** the second anionic surfactant is a sarcosinate surfactant according to the general formula 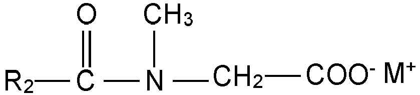 wherein R₂ is a saturated or unsaturated, straight or branched alkyl chain with 7 to 17 C atoms, and preferably 9 to 13 C atoms, and M is H, sodium, potassium, ammonium or ammonium.

5. The composition according to any of the preceding claims **characterized in that** the second anionic surfactant is a glutamate surfactant according to the general formula 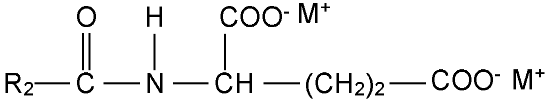 wherein R₂ is a saturated or unsaturated, straight or branched alkyl chain with 7 to 17 C atoms, and more preferably 9 to 13 C atoms, and M is independent from each other H, sodium, potassium or ammonium.

6. The composition according to any of the preceding claims **characterized in that** the non-ionic surfactant is an alkyl (poly)glucoside according to the general formula
R₈-O-(R₉O)ₙ-Zₓ,
wherein R₈ is an alkyl group with 8 to 18 carbon atoms, R₉ is an ethylene or propylene group, Z is a saccharide group with 5 to 6 carbon atoms, n is a number from 0 to 10 and x is a number between 1 and 5.

7. The composition according to any of the preceding claims **characterized in that** it comprises anionic surfactants at a concentration in the range of 50 to 95% by weight of the total surfactants.

8. The composition according to any of the preceding claims **characterized in that** it comprises as the first anionic surfactant one surfactant selected from laureth, myreth and coceth sulphates or their salts such as sodium, potassium or ammonium salts, preferably it is sodium laureth sulphates with 1.5 to 3 ethoxy units on average.

9. The composition according to any of the preceding claims **characterised in that** it comprises the first anionic surfactant at a concentration of 30 to 70%, by weight calculated to the total anionic surfactants content of the composition.

10. The composition according to any of the preceding claims **characterized in that** it comprises the second sarcosinate surfactant and the third glutamate surfactants at a weight ratio of the sarcosinate to glutamate surfactant in the range of 1:1 to 10:1, preferably 1:1 to 8:1 and more preferably 1:1 to 6:1 and most preferably 1:1 to 5:1 and in particular in the range of 3:1 to 4:1.

11. The composition according to any of the preceding claims **characterised in that** it comprises non-ionic surfactant at a concentration in the range of 5 to 80%, preferably 5 to 70%, more preferably 5 to 60% and most preferably 5 to 50% by weight of the total surfactants.

12. The composition according to any of the preceding claims **characterized in that** it comprises one or more of the ingedients seleceted from
- cationic polymer,
- hair dye, especially direct dye,
- fatty alcohol,
- organic solvent,
- thickening agent,
- pearlizing agent,
- fragrance,
- Coenzyme, and
- UV filter

13. Use of a composition according to claims 1 to 12 for cleansing hair, especially artificially coloured hair.

14. Kit for treating hair comprising a cleansing composition according to claims 1 to 12 and optionally at least one composition comprising a hair dye.

## Patentansprüche

1. Wässrige Reinigungszusammensetzung für Keratinfasern, insbesondere für menschliche Haare, **dadurch gekennzeichnet, dass** sie drei anionische Tenside aufweist, wobei das erste anionische Tensid ein Alkylethersulfat-Tensid gemäß der folgenden allgemeinen Struktur
R₁ (OCH₂CH₂)ₙ OSO₃ M
ist, wobei R₁ für eine gesättigte oder ungesättigte, verzweigte oder lineare Alkylkette mit 10 bis 18 C-Atomen steht, n eine Zahl zwischen 1 und 5 ist (die Werte sind eingeschlossen) und M für ein monovalentes Kation steht, vorzugsweise ausgewählt aus H, Ammonium, Natrium und Kalium, und das zweite und das dritte anionische Tensid Aminosäure-Tenside sind, ausgewählt aus der folgenden allgemeinen Struktur 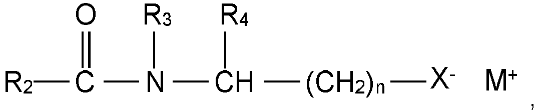 wobei R₂ für eine gesättigte oder ungesättigte, lineare oder verzweigte Alkylkette mit 7 bis 17 C-Atomen steht, R₃ für H oder ein Methyl steht, R₄ für H, COO⁻ M⁺, CH₂COO⁻ M oder COOH steht, n 0 bis 2 beträgt, X für COO⁻ oder SO₃⁻ steht und M unabhängig voneinander für H, Natrium, Kalium oder Ammonium steht, und ein Alkyl(poly)glucosid als ein nichtionisches Tensid aufweist, wobei sie frei von amphoteren Tensiden ist und die Tensid-Gesamtkonzentration im Bereich von 7,5 Gewichts-% bis 50 Gewichts-% liegt, bezogen auf die Gesamtzusammensetzung.

2. Zusammensetzung nach Anspruch 1, **dadurch gekennzeichnet, dass** das erste anionische Tensid aus Natriumlaurethsulfaten mit durchschnittlich 1,5 bis 3 Ethoxy-Einheiten ausgewählt ist.

3. Zusammensetzung nach Anspruch 1 und 2, **dadurch gekennzeichnet, dass** die Konzentration des ersten Sulfat-Tensids im Bereich von 20 Gewichts-% bis 80 Gewichts-% liegt, bezogen auf die gesamten anionischen Tenside.

4. Zusammensetzung nach einem der vorhergehenden Ansprüche, **dadurch gekennzeichnet, dass** das zweite anionische Tensid ein Sarcosinat-Tensid gemäß der allgemeinen Formel 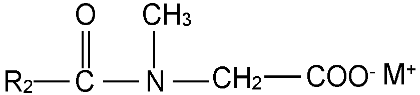 ist, wobei R₂ für eine gesättigte oder ungesättigte, lineare oder verzweigte Alkylkette mit 7 bis 17 C-Atomen, vorzugsweise 9 bis 13 C-Atomen, steht und M für H, Natrium, Kalium, Ammonium oder Ammonium steht.

5. Zusammensetzung nach einem der vorhergehenden Ansprüche, **dadurch gekennzeichnet, dass** das zweite anionische Tensid ein Glutamat-Tensid gemäß der allgemeinen Formel 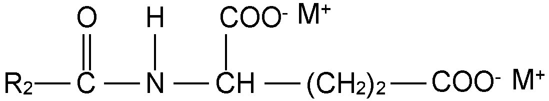 ist, wobei R₂ für eine gesättigte oder ungesättigte, lineare oder verzweigte Alkylkette mit 7 bis 17 C-Atomen, vorzugsweise 9 bis 13 C-Atomen, steht und M unabhängig voneinander für H, Natrium, Kalium oder Ammonium steht.

6. Zusammensetzung nach einem der vorhergehenden Ansprüche, **dadurch gekennzeichnet, dass** das nichtionische Tensid ein Alkyl(poly)glucosid ist, gemäß der allgemeinen Formel
R₈-O-(R₉O)ₙ-Zₓ,
wobei R₈ für eine Alkylgruppe mit 8 bis 18 Kohlenstoffatomen steht, R₉ für eine Ethylen- oder Propylengruppe steht, Z für eine Saccharidgruppe mit 5 bis 6 Kohlenstoffatomen steht, n eine Zahl von 0 bis 10 ist und x eine Zahl zwischen 1 und 5 ist.

7. Zusammensetzung nach einem der vorhergehenden Ansprüche, **dadurch gekennzeichnet, dass** sie anionische Tenside in einer Konzentration im Bereich von 50 Gewichts-% bis 95 Gewichts-% der Gesamttenside aufweist.

8. Zusammensetzung nach einem der vorhergehenden Ansprüche, **dadurch gekennzeichnet, dass** sie als das erste anionische Tensid ein Tensid aufweist, das aus Laureth-, Myreth- und Cocethsulfaten oder deren Salzen, wie z.B. Natrium-, Kalium- oder Ammoniumsalzen, ausgewählt ist, es sich vorzugsweise um Natriumlaurethsulfate mit durchschnittlich 1,5 bis 3 Ethoxyeinheiten handelt.

9. Zusammensetzung nach einem der vorhergehenden Ansprüche, **dadurch gekennzeichnet, dass** sie das erste anionische Tensid in einer Konzentration von 30 Gewichts-% bis 70 Gewichts-% aufweist, bezogen auf den Gesamtgehalt an anionischen Tensiden der Zusammensetzung.

10. Zusammensetzung nach einem der vorhergehenden Ansprüche, **dadurch gekennzeichnet, dass** sie das zweite Sarcosinat-Tensid und das dritte Glutamat-Tensid in einem Gewichtsverhältnis des Sarcosinat-Tensids zum Glutamat-Tensid im Bereich von 1:1 bis 10:1, vorzugsweise 1:1 bis 8:1 und mehr bevorzugt 1:1 bis 6:1 und am meisten bevorzugt 1:1 bis 5:1 und insbesondere im Bereich von 3:1 bis 4:1 aufweist.

11. Zusammensetzung nach einem der vorhergehenden Ansprüche, **dadurch gekennzeichnet, dass** sie nichtionisches Tensid in einer Konzentration im Bereich von 5 Gewichts-% bis 80 Gewichts-%, vorzugsweise 5 Gewichts-% bis 70 Gewichts-%, mehr bevorzugt 5 Gewichts-% bis 60 Gewichts-% und am meisten bevorzugt 5 Gewichts-% bis 50 Gewichts-% der Gesamttenside aufweist.

12. Zusammensetzung nach einem der vorhergehenden Ansprüche, **dadurch gekennzeichnet, dass** sie einen oder mehrere der Inhaltsstoffe aufweist, ausgewählt aus
- kationischem Polymer,
- Haarfarbstoff, insbesondere Direktfarbstoff,
- Fettalkohol,
- organischem Lösungsmittel,
- Verdickungsmittel,
- Perlglanzmittel,
- Duftstoff,
- Coenzym und
- UV-Filter.

13. Verwendung einer Zusammensetzung nach Anspruch 1 bis 12 zum Reinigen von Haaren, insbesondere künstlich gefärbten Haaren.

14. Kit zum Behandeln von Haaren, welches eine Reinigungszusammensetzung nach Anspruch 1 bis 12 und gegebenenfalls mindestens eine Zusammensetzung aufweist, welche einen Haarfarbstoff aufweist.

## Revendications

1. Composition de nettoyage aqueuse pour fibres de kératine, notamment pour des cheveux humains, **caractérisée en ce que** elle comprend trois tensioactifs anioniques, où le premier tensioactif anionique est un tensioactif de type alkyl éther sulfate selon la structure générale suivante
R₁ (OCH₂CH₂)ₙ OSO₃ M
où R₁ est une chaîne alkyle saturée ou insaturée, ramifiée ou linéaire, avec 10 à 18 atomes C, n est un nombre entre 1 à 5 (les valeurs sont incluses) et M est un cation monovalent, de préférence choisi parmi un H, un ammonium, un sodium et un potassium, et
le deuxième et le troisième tensioactif anioniques sont des tensioactifs de type acide aminé choisis à partir de la structure générale suivante 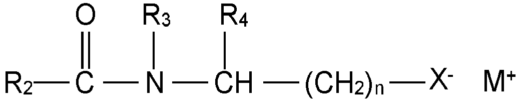
où R₂ est une chaîne alkyle saturée ou insaturée, linéaire ou ramifiée, avec de 7 à 17 atomes C, R₃ est un atome H ou un groupe méthyle, R₄ est un atome H, un groupe COO⁻ M⁺, un groupe CH₂COO⁻ M ou COOH, n est égal à un nombre de 0 à 2, X est un groupe COO⁻ ou SO₃⁻ et les M sont indépendamment les uns des autres un ion H, un ion sodium, un ion potassium ou ammonium, et un alkyl (poly)glucoside comme un tensioactif non ionique, où elle est exempte de tensioactifs amphotères et la concentration en tensioactifs totale est dans la plage de 7,5 à 50 % en poids, calculée par rapport à la composition totale.

2. Composition selon la revendication 1, **caractérisée en ce que** le premier tensioactif anionique est choisi parmi des lauryl éthers sulfates de sodium avec de 1,5 à 3 unités éthoxy en moyenne.

3. Composition selon les revendications 1 et 2, **caractérisée en ce que** la concentration du premier tensioactif de type sulfate est dans la plage de 20 à 80 % en poids, calculée par rapport au total des tensioactifs anioniques.

4. Composition selon l'une quelconque des revendications précédentes, **caractérisée en ce que** le deuxième tensioactif anionique est un tensioactif de type sarcosinate selon la formule générale 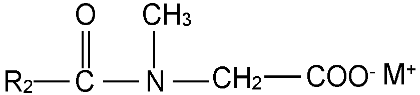 où R₂ est une chaîne alkyle saturée ou insaturée, linéaire ou ramifiée, avec de 7 à 17 atomes C, et de préférence 9 à 13 atomes C, et M est un ion H, sodium, potassium, ammonium ou ammonium.

5. Composition selon l'une quelconque des revendications précédentes, **caractérisée en ce que** le deuxième tensioactif anionique est un tensioactif de type glutamate selon la formule générale 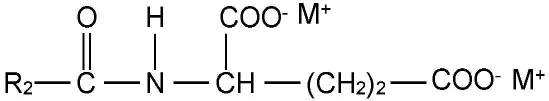 où R₂ est une chaîne alkyle saturée ou insaturée, linéaire ou ramifiée, avec de 7 à 17 atomes C, et plus préférentiellement de 9 à 13 atomes C, et les M sont indépendamment les uns des autres un ion, sodium, potassium ou ammonium.

6. Composition selon l'une quelconque des revendications précédentes, **caractérisée en ce que** le tensioactif non ionique est un alkyl (poly)glucoside, selon la formule générale
R₈-O-(R₉O)ₙ-Zₓ,
où R₈ est un groupe alkyle avec de 8 à 18 atomes de carbone, R₉ est un groupe éthylène ou propylène, Z est un groupe saccharide avec de 5 à 6 atomes de carbone, n est un nombre de 0 à 10 et x est un nombre entre 1 et 5.

7. Composition selon l'une quelconque des revendications précédentes, **caractérisée en ce qu'**elle comprend des tensioactifs anioniques à une concentration dans la plage de 50 à 95 % en poids du total des tensioactifs.

8. Composition selon l'une quelconque des revendications précédentes, **caractérisée en ce qu'**elle comprend en tant que premier tensioactif anionique un tensioactif choisi parmi les lauryl éther, myreth et coceth sulfates ou leurs sels, tels que les sels de sodium, de potassium ou d'ammonium, il s'agit de préférence de lauryl éther sulfate de sodium avec de 1,5 à 3 unités éthoxy en moyenne.

9. Composition selon l'une quelconque des revendications précédentes, **caractérisée en ce qu'**elle comprend le premier tensioactif anionique à une concentration de 30 à 70 % en poids, calculée par rapport aux tensioactifs anioniques totaux de la com position.

10. Composition selon l'une quelconque des revendications précédentes, **caractérisée en ce qu'**elle comprend le deuxième tensioactif de type sarcosinate et le troisième tensioactif de type glutamate dans un ratio en poids de tensioactif de type sarcosinate sur tensioactif de type glutamate dans la plage de 1 : 1 à 10 : 1, de préférence de 1 : 1 à 8 : 1, et plus préférentiellement de 1 : 1 à 6 : 1, et idéalement de 1 : 1 à 5 : 1, et en particulier dans la plage de 3 : 1 à 4 : 1.

11. Composition selon l'une quelconque des revendications précédentes, **caractérisée en ce qu'**elle comprend le tensioactif non ionique à une concentration dans la plage de 5 à 80 %, de préférence de 5 à 70 %, plus préférentiellement de 5 à 60 % et idéalement de 5 à 50 % en poids des tensioactifs totaux.

12. Composition selon l'une quelconque des revendications précédentes, **caractérisée en ce qu'**elle comprend un ou plusieurs ingrédients choisis parmi
- un polymère cationique,
- un colorant pour cheveux, notamment un colorant direct,
- un alcool gras,
- un solvant organique,
- un agent épaississant,
- un agent perlant,
- un parfum,
- une coenzyme, et
- un filter UV

13. Utilisation d'une composition selon les revendications 1 à 12 pour le nettoyage des cheveux, notamment de cheveux colorés artificiellement.

14. Kit pour traiter les cheveux comprenant une composition de nettoyage selon les revendications 1 à 12 et éventuellement au moins une composition comprenant un colorant pour cheveux.
